# EUROPÄISCHE PATENTSCHRIFT

(11) **EP 0 687 502 B1**
(45) Veröffentlichungstag und Bekanntmachung des Hinweises auf die Patenterteilung: **12.03.2003**
(21) Anmeldenummer: 95108929.1
(22) Anmeldetag: 09.06.1995
(51) Int. Cl.: B01L 7/00, C12Q 1/68, G01N 1/28

(54) **Verfahren und Vorrichtung zur Behandlung von Nukleinsäuren aus einer Probe**
Method and device for treating nucleic acids from a sample
Procédé et dispositif pour le traitement d'acides nucléiques dans un échantillon

(30) Priorität: 15.06.1994 DE 4420732
(43) Veröffentlichungstag der Anmeldung: 20.12.1995
(73) Patentinhaber: Roche Diagnostics GmbH, 68298 Mannheim (DE)
(72) Erfinder: Bienhaus, Gerhard, Dr., D-82407 Wielenbach (DE); Lange, Hans, D-68623 Lampertheim (DE)

(56) Entgegenhaltungen:
- WO-A-93/13220
- WO-A-93/22020
- WO-A-93/22058
- WO-A-94/25785
- FR-A- 2 672 301

## Beschreibung

Gegenstand der Erfindung ist ein Verfahren und eine Vorrichtung zur Behandlung von Nukleinsäuren aus einer Probe sowie ein System zur Handhabung dieser Vorrichtung, nach den Ansprüchen 1, 12 und 13.

Im Gegensatz zur klinischen Chemie und Immunologie spielt bei der Analyse von Nukleinsäuren die Probenvorbereitung eine entscheidende Rolle, vor allem, wenn Amplifikationsverfahren wie die Polymerase Chain Reaction (US-A-4,683,195) oder auf Transkriptionsreaktionen basierende Amplifikationen Teil des Analyseverfahrens sind. Dabei kann ein Nukleinsäureanalyseverfahren theoretisch in drei Teilschritte zerlegt werden, nämlich eine Probenvorbereitung, in der die Nukleinsäuren aus einer Probe zugänglich gemacht und von störenden Probenbestandteilen abgetrennt werden, eine Amplifikation, bei der die Menge an Nukleinsäuren erhöht wird, und einen Detektionsschritt, in dem die gebildeten Nukleinsäuren nachgewiesen werden.

In derzeit erhältlichen Nukleinsäureanalysetests, z. B. für den Nachweis von HIV aus Blut, sind eine ganze Reihe von chemischen und physikalischen Behandlungsschritten erforderlich. Dazu gehören Zentrifugations-, Dekantierungs- und Extraktionsschritte. Diese Schritte finden in Reagenzröhrchen statt. Ergebnis der Probenvorbereitung sind gefällte Nukleinsäuren, die für den Einsatz in der Amplifikationsreaktion wieder aufgelöst werden.

Eine spezielle Variante, bei der die Nukleinsäuren nach der Probenvorbereitung in an ein Gel gebundender Form vorliegen, ist in der EP-A-0 389 063 beschrieben. Auch dieses Verfahren beinhaltet mehrere Zentrifugationsschritte zur Abtrennung der Gelpartikel von der Probenflüssigkeit.

Ein besonderes Problem bei der Analyse von Proben auf Nukleinsäuren ist die Gefahr einer Kontamination durch von außen, über die Luft oder mit Bearbeitungsvorrichtungen eingeschleppte Nukleinsäuren. Die oben geschilderten Verfahren sind zusätzlich noch besonders kontaminationsanfällig, da die Probengläschen mehrmals geöffnet und geschlossen werden müssen.

In EP-B-0 381 501 ist eine Vorrichtung zur Vermehrung von Nukleinsäuren und zur Detektion der Vermehrungsprodukte beschrieben, bei der eine Probe in eine Reaktionskammer zur Vermehrung pipettiert wird und die Flüssigkeit nach Durchführung der Vermehrung unter Ausschluß eines Kontaktes mit der Umgebung in eine Detektionskammer überführt wird. Während diese Vorrichtung zur Vermeidung des Austritts von Kontaminationen während Vermehrung und Detektion geeignet sein mag, läßt die EP-B-0 381 501 völlig außer acht, daß in der praktischen Diagnostik Nukleinsäuren einer Vorbereitung bedürfen, da sie üblicherweise nicht in direkt in die PCR einsetzbarer Form vorliegen.

Das Dokument WO-A-9322058 beschreibt eine Vorrichtung zur behandlung von Nukleinsäuren, bei der in Serie geschaltete Reaktionsräume mit einem Filter zum Zurückhalten unerwünschter Bestandteile verwendet werden.

Aufgabe der vorliegenden Erfindung war es daher, Nachteile des Standes der Technik zu vermeiden und insbesondere eine Vorrichtung zur kontaminationsarmen und einfachen Behandlung von Nukleinsäuren bereitzustellen.

Gegenstand der Erfindung ist daher ein Verfahren und eine Vorrichtung zur Behandlung von Nukleinsäuren aus eine Probe nach den Ansprüchen 1 und 12. Ebenfalls Gegenstand der Erfindung ist ein System zur Behandlung von Nukleinsäuren, welches auf die erfindungsgemäße Vorrichtung abgestimmt ist.

Grundgedanke der Erfindung ist, daß die Teilschritte Probenvorbereitung und Vermehrung von Nukleinsäuren besonders vorteilhaft aneinander gekoppelt in einer einzigen Vorrichtung durchgeführt werden. Eine solche Kopplung der Teilschritte wurde bisher noch nicht beschrieben.

Nukleinsäuren im Sinne der Erfindung sind jegliche Art von Nukleinsäuren, sowohl DNA als auch RNA, einzelsträngig, doppelsträngig oder mehrfachsträngig. Diese Nukleinsäuren können in jeder Art von Proben, insbesondere flüssigen Proben, enthalten sein. Unter Proben ist hierbei ein Gemisch von Stoffen zu verstehen. Sie können vor ihrem Einsatz in der erfindungsgemäßen Vorrichtung einer Behandlung unterzogen worden sein, können jedoch auch der Natur direkt entnommen sein, z. B. Körperflüssigkeiten wie Blut oder Urin.

Eine Behandlung von Nukleinsäuren ist für die Durchführung verschiedenster Verfahren erforderlich. Hierzu gehören Verfahren zum Nachweis von Nukleinsäuren in einer Probe, wie sie bei der Erkennung von Infektionen durch nukleinsäurehaltige Organismen erforderlich sind. Mehrere Behandlungsschritte sind jedoch auch in Verfahren zur Feststellung des genetischen Status von Organismen sinnvoll. In all diesen Verfahren sind eine Reihe von Behandlungsschritten erforderlich. Da die Nukleinsäuren nur einen sehr geringen Anteil der Komponenten des Gemisches ausmachen, wird oft eine Abtrennung von Nukleinsäuren von anderen Probenbestandteilen vorgenommen. Der angestrebte Grad der Abtrennung bzw. Aufreinigung richtet sich nach dem beabsichtigten Zweck der Gesamtbehandlung.

Die Vermehrung von Nukleinsäuren ist ebenfalls ein weit verbreiteter Schritt bei der Behandlung von Nukleinsäuren. Es stehen diverse Verfahren zur Vermehrung von Nukleinsäuren bereit, z. B. die Polymerasekettenreaktion. Unter Vermehrung von Nukleinsäuren ist im wesentlichen die in-vitro-Vermehrung gemeint. Jedoch ist auch eine in-vivo-Vermehrung prinzipiell denkbar.

Gegenstand der Erfindung ist nun die Verknüpfung beider Behandlungsschritte in einer einzigen Vorrichtung. Dabei findet die Abtrennung der Nukleinsäuren von anderen Probenbestandteilen in einem ersten Reaktionsraum statt und die Vermehrung der Nukleinsäuren in einem zweiten Reaktionsraum. Erfindungsgemäß sind die beiden Reaktionsräume über einen Transportweg miteinander verbunden, wobei dieser so gestaltet ist, daß er eine zeitliche Festlegung des Transports einer Flüssigkeit vom ersten in den zweiten Reaktionsraum erlaubt.

Der Behandlungsschritt zum Abtrennen der Nukleinsäuren im ersten Reaktionsraum ist ein komplexer Vorgang, der je nach Probenart folgende Unterschritte beinhalten kann, die auch in mehr als einem erfindungsgemäß verbundenen Reaktionsraum ablaufen können. Er kann beispielsweise beinhalten:
a) Verflüssigung im Falle einer festen oder zähflüssigen Probe.
b) Vereinzelung von nukleinsäureenthaltenden Kompartimenten wie z. B. Zellen oder Zellkompartimente über geeignete, großteils bekannte Verfahren.
c) Anreicherung von nukleinsäureenthaltenden Kompartimenten wie z. B. Zellen oder Zellkompartimenten über geeignete Verfahren (z. B. gemäß EP-A-0260280).
d) Freisetzung von Nukleinsäuren aus den obenerwähnten Kompartimenten.
e) Die Abtrennung von Nukleinsäuren von anderen Probenbestandteilen, vor allem solchen, die in den verschiedensten Vermehrungsprozessen inhibitorisch wirken, z. B. Polymeraseinhibitoren.

All diese Behandlungsschritte im ersten Reaktionsraum geschehen erfindungsgemäß nicht über Zentrifugationsschritte. Möglichkeiten dafür sind als Verfahren in der Literatur beschrieben und beinhalten enzymatische Abbaureaktionen, diverse immunchemische Verfahren mit verschiedensten Festphasen-Systemen wie Immunadsorption oder Einsatz von beschichteten Magnetpartikeln. Möglichkeiten zur Abtrennung von Nukleinsäuren sind gegeben durch Zurückhalten der Nukleinsäuren an einem Filtermaterial, (unspezifische) Immobilisierung von Nukleinsäuren an ein im Reaktionsraum enthaltenes Adsorbens wie beispielsweise Silikagel oder Glaspartikel, oder biospezifische Immobilisierung von Nukleinsäuren an biospezifischen Adsorbentien, z. B. Affinitätsmaterialien. Hierbei kann die Fähigkeit von Nukleinsäuren, mit hierzu im wesentlichen komplementären Nukleinsäuren an einer festen Phase Hybride zu bilden, ausgenutzt werden.

Die Abtrennung der Nukleinsäuren kann auch über chromatographische Verfahren im ersten Reaktionsraum vorgenommen werden. Neben der Abtrennung von Nukleinsäuren können im ersten Reaktionsraum zusätzlich weitere Behandlungsschritte für Nukleinsäuren durchgeführt werden, z. B. Filtration von nukleinsäurehaltigen Zellen, Lyse dieser Zellen oder Schritte zur Markierung von Nukleinsäuren. Beispielsweise kann im ersten Reaktionsraum gemäß der Erfindung in einem Schritt die Lyse und Immobilisierung von Nukleinsäuren gemäß EP-A-0 389 063 durchgeführt werden. Je nach den im ersten Reaktionsraum durchzuführenden Behandlungen, kann auch, angrenzend an den Reaktionsraum, ein Heizelement vorgesehen sein. Dieses ist insbesondere bei der Lyse von Zellen praktikabel.

Da einerseits bei allen hier gezeigten Verfahren vor einer Nukleinsäurevermehrung die Kontaminationsgefahr sehr groß ist und ein hohes Risiko birgt, falsch-positive Ergebnisse zu erzielen und andererseits wie beschrieben die Prozesse für die entsprechende Probenaufarbeitung sehr unterschiedlicher Natur sind, ist ein wichtiger Gegenstand der Erfindung die Verwendung von kostengünstigen modular anwendbaren Einmalplastikartikeln, die in einem weitgehend geschlossenem Verbund für Mehrstufenprozesse benutzt werden. Die Vorrichtung stellt daher ein im wesentlichen geschlossenes System dar, das an wenigen Stellen ohne Kontaminationsrisiko geöffnet werden kann, um mindestens einen Reagenzeintrag in mindestens einen Reaktionsraum zu ermöglichen. In einem ersten Aspekt der Erfindung stellt die Vorrichtung daher ein anwendungsbereites Mehrfachreaktionsgefäß dar.

Die Abtrennung der Nukleinsäure von anderen Probenbestandteilen wird beendet, indem die Flüssigkeit mit anderen Probenbestandteilen aus dem ersten Reaktionsraum entfernt wird, wohingegen die angereicherten Nukleinsäuren im Reaktionsraum verbleiben. Dazu weist der erste Reaktionsraum neben der für das Einfüllen der Probe in den ersten Reaktionsraum erforderlichen Öffnung eine weitere Öffnung bevorzugt am unteren Teil oder am Boden des Reaktionsraumes auf, die im weiteren als Ventilöffnung bezeichnet wird. Diese Ventilöffnung ist bevorzugt verschließbar. Sobald die Probe in dem ersten Reaktionsraum vorliegt, ist der Verschluß normalerweise geschlossen. Zur Abtrennung der anderen Probenbestandteile von den Nukleinsäuren wird der Verschluß, solange erforderlich, geöffnet und die anderen Probenbestandteile entnommen.

Als Verschluß im Sinne der Erfindung hat sich dafür insbesondere ein Ventilverschluß erwiesen, der durch Anlegen eines Vakuums von außen an den ersten Reaktionsraum geöffnet werden kann. Durch Anlegen eines Unterdrucks gegenüber dem im ersten Reaktionsraum vorherrschenden Druck werden die Probenbestandteile aus dem ersten Reaktionsraum entfernt. Wenn diese nicht mehr weiterbearbeitet werden sollen, werden sie verworfen, bevorzugt in ein Abfallgefäß transportiert.

Da die Öffnung zum Befüllen des Reaktionsraumes, die sich bevorzugt im oberen Teil des Reaktionsraumes befindet, wegen der hohen Kontaminationsgefahr mit einem geeigneten automatisch zu öffnenden Deckel verschlossen sein muß, wie er in DE-A-4412286 beschrieben ist, muß für einen angemessenen Druckausgleich Sorge getragen werden. Erfindungsgemäße Lösungen werden später beschrieben.

Anschließend werden die vorbereiteten, z. B. von anderen Probenbestandteilen abgetrennten Nukleinsäuren in einen zweiten Reaktionsraum überführt, in dem die Vermehrung der Nukleinsäuren stattfindet. Zwischen dem ersten und zweiten Reaktionsraum können jedoch noch weitere Reaktionsräume zur weiteren Aufreinigung oder für andere Behandlungsschritte vorgesehen sein. Die Nukleinsäuren werden aus dem ersten Reaktionsraum durch Lösen in einer Flüssigkeit entfernt. Je nach Art der Immobilisierung der Nukleinsäuren kann dies durch Anlegen eines Reagenzgradienten, eines die Desorption bewirkenden Stoffes oder eine Verdrängung geschehen. Geeignete Verfahren sind insbesondere aus der EP-A-0 389 063 bekannt.

Die die Nukleinsäuren enthaltende Flüssigkeit wird über einen Transportweg zum zweiten Reaktionsraum transportiert. Erfindungsgemäß bevorzugt ist dieser Transportweg regelbar. Unter regelbar wird verstanden, daß der Transportweg Mittel enthält, mit denen sichergestellt wird, daß nur die nukleinsäurehaltige Lösung und nicht die mit anderen Probenbestandteilen beladene Flüssigkeit in den zweiten Reaktionsraum gelangt. Dies wird realisiert durch einen konventionellen 3-Wege-Hahn, von dem jeweils ein Weg zu dem ersten und zweiten Reaktionsraum führt und ein dritter Weg ein Abfalltransportweg ist. Wenn die Flüssigkeit mit den anderen Probenbestandteilen durch den Transportweg transportiert werden soll, verbindet das Verteilerelement den vom ersten Reaktionsraum kommenden Weg mit dem Abfalltransportweg. Wenn die nukleinsäurehaltige Lösung in den zweiten Reaktionsraum überführt werden soll, wird das Verteilerelement so gestellt, daß der Weg vom ersten Reaktionsraum mit dem Weg zum zweiten Reaktionsraum verbunden wird. Weitere Möglichkeiten zur Teilung der Flüssigkeitsströme sind bekannt. Das Verteilerelement wird bevorzugt über eine zentrale Steuereinheit gesteuert, so daß die Zugabe von Reagenzien in den ersten Reaktionsraum, die Initiation des Transports der Flüssigkeit mit den anderen Probenbestandteilen bzw. der nukleinsäurehaltigen Lösung und die Stellung des Verteilerelements koordiniert werden kann.

Der zweite Reaktionsraum, welcher für die Durchführung der Vermehrungsreaktion benutzt wird, enthält die hierfür erforderlichen Reagenzien entweder bereits vor der Zugabe der nukleinsäurehaltigen Lösung oder diese Reagenzien werden über eine Öffnung zu der nukleinsäurehaltigen Flüssigkeit im zweiten Reaktionsraum zugegeben. Bevorzugt ist die hierfür vorgesehene Öffnung zur Verringerung der Kontaminationsgefahr von außen verschließbar und wird nach Herstellung der Vermehrungsbedingungen verschlossen. Sofern die Zugabe weiterer Reagenzien zu einem späteren Zeitpunkt erforderlich wird, kann die Öffnung erneut geöffnet werden. Eine weit verbreitete Möglichkeit der Vermehrung von Nukleinsäuren ist die PCR, z. B. nach EP-A-0 200 362. Der zweite Reaktionsraum ist dazu bevorzugt so gestaltet, daß schnelle Temperaturänderungen darin vollzogen werden können.

Nach der Vermehrungsreaktion kann die Reaktionsmischung für die Durchführung weiterer Reaktionen in dem zweiten Reaktionsraum verbleiben, oder aber daraus entfernt werden. Hierfür steht entweder die oben genannte verschließbare Öffnung zur Verfügung oder es kann eine weitere Öffnung, wie für den ersten Reaktionsraum beschrieben, vorgesehen sein.

Für die Durchführung von Nukleinsäurenachweistests kann der Nachweis im zweiten Reaktionsraum geschehen, beispielsweise wie in der DE-A-43 44 742 beschrieben, oder nach Überführung in ein weiteres Gefäß, beispielsweise einen dritten Reaktionsraum.

Die einzelnen Elemente der erfindungsgemäßen Vorrichtung sind prinzipiell bekannt bzw. sehr leicht herstellbar. Sie sind beispielsweise Tubes, die an ihrem Boden ein durch Unterdruck zu öffnendes Ventil enthalten, indem eine in der Mitte geschlitzte Gummidichtlippe sich in Richtung des Unterdruckes wölbt und die außenliegenden Auslaßöffnungen des Tubebodens freigibt. Wird der Unterdruck aufgehoben, so springt die Gummidichtlippe in die Ausgangsposition zurück und dichtet die Auslaßöffnungen.

Der Tubekörper kann aus geeigneten Materialien, wie beispielsweise Kunststoffen oder Glas, hergestellt sein. Die Gummidichtlippe kann aus elastischem Kautschukgummi oder vergleichbar elastischen Material hergestellt werden, wobei eine hohe Materialelastizität eine Öffnung durch geringen Unterdruck und umgekehrt ermöglicht.

Die Herstellung erfolgt in zwei Schritten: a.) Spritzgußtechnische Verarbeitung von Kunststoffen wie oben erwähnt zur Formgebung eines Grundkörpers, der am unteren Ende einen Rand überstehen hat. b.) Einlegen der Gummidichtlippe mit anschließender Umbördelung des Randes mittels Schmelzen, wodurch die Befestigung der Gummidichtlippe an den Grundkörper entsteht.

Der regelbare Transportweg kann aus einem Stück Schlauch bestehen, jedoch auch aus einer festen Röhre aus Kunststoff oder Plastik bestehen. Als Verteilerelement können handelsübliche 3-Wege-Verteilerelemente eingesetzt werden. Als zweiter Reaktionsraum kann praktisch jedes Tube, wie es aus Reagenzienautomaten bekannt sind, verwendet werden. Besonders vorteilhaft ist die erfindungsgemäße Vorrichtung jedoch aus einem Stück gebildet, mit Ausnahme der bewegten Teile, wie Ventil oder Verteiler. Zur Herstellung stehen Spritzgußverfahren zur Verfügung.

Ein Vorteil der erfindungsgemäßen Vorrichtung ist, daß sie als Einmal-Device gestaltet werden kann, d. h. nach Abtrennung der Nukleinsäuren und ihrer Vermehrung sowie Gewinnung der vermehrten Nukleinsäuren kann die Vorrichtung verworfen werden.

Eine weitere mögliche Ausführungsform der erfindungsgemäßen Vorrichtung sieht vor, daß die Vorrichtung aus einzelnen Modulen aufgebaut ist, die je nach der Art der durchzuführenden Behandlungsschritte, die von der Art der Probe und der gewünschten Behandlung abhängig ist, aus einzelnen Modulen beliebig zusammengesetzt werden kann. Unter einem Modul wird eine Vorrichtung verstanden, die für einen Behandlungsschritt vorgesehen ist, und die mit anderen Vorrichtungen zur Abarbeitung einer ganzen Kaskade von Behandlungsschritten auf einfache Weise verbunden und kombiniert werden kann. In einem Aspekt der Erfindung besteht die erfindungsgemäße Vorrichtung aus im wesentlichen baugleichen, zusammensteckbaren Modulen, so daß eine mehrstufige Reaktionsführung möglich ist. Im wesentlichen baugleich bedeutet hier, daß mindestens zwei Module eine ähnliche äußere Form und ein gleiches Prinzip für die Öffnung des Verschlusses, mit dem die Entnahmeöffnung verschlossen werden kann, aufweist. Im Hinblick auf die Inhalte des Reaktionsraumes des Moduls können je nach Einsatzzweck des Moduls beträchtliche Unterschiede bestehen, z. B. kann ein Modul ein Filtervlies, ein anderes Reagenzien zur Lyse von Zellen, ein weiteres Materialien zur Immobilisierung von Zellen oder Nukleinsäuren enthalten. Der Begriff zusammensteckbar bedeutet, daß der Ausgang eines Moduls mit einer Eingangsöffnung eines anderen Moduls schnell flüssigkeitsdicht verbunden werden kann. Als Module können Module verwendet werden, die einen Reaktionsraum sowie einen flüssigkeitsdichten Verschluß, der durch Einwirkung von Vakuum von außen auf den Verschluß geöffnet werden kann, aufweisen. Für die Abtrennung der Nukleinsäuren enthält das Modul die für die Abtrennung erforderlichen Materialien und Reagenzien, während für die Durchführung der Vermehrungsreaktion die vermehrungsreaktionsspezifischen Reagenzien enthalten sind. Die Reaktionsmodule können über Verteilermodule miteinander verbunden werden. Die Verteilermodule enthalten Ansätze für die Verbindung mit den Reaktionsmodulen und einem Abfallgefäß, die Transportwege und das Verteilerelement.

Der Transport der Gase und insbesondere Flüssigkeiten innerhalb der Vorrichtung wird entweder über Kapillarität oder Unterdruck/Vakuum gewährleistet. Bevorzugt ist die Benutzung von Unterdruck. Hierzu wird an den Abfallbehälter und/oder den zweiten Reaktionsraum ein leichter Unterdruck angelegt.

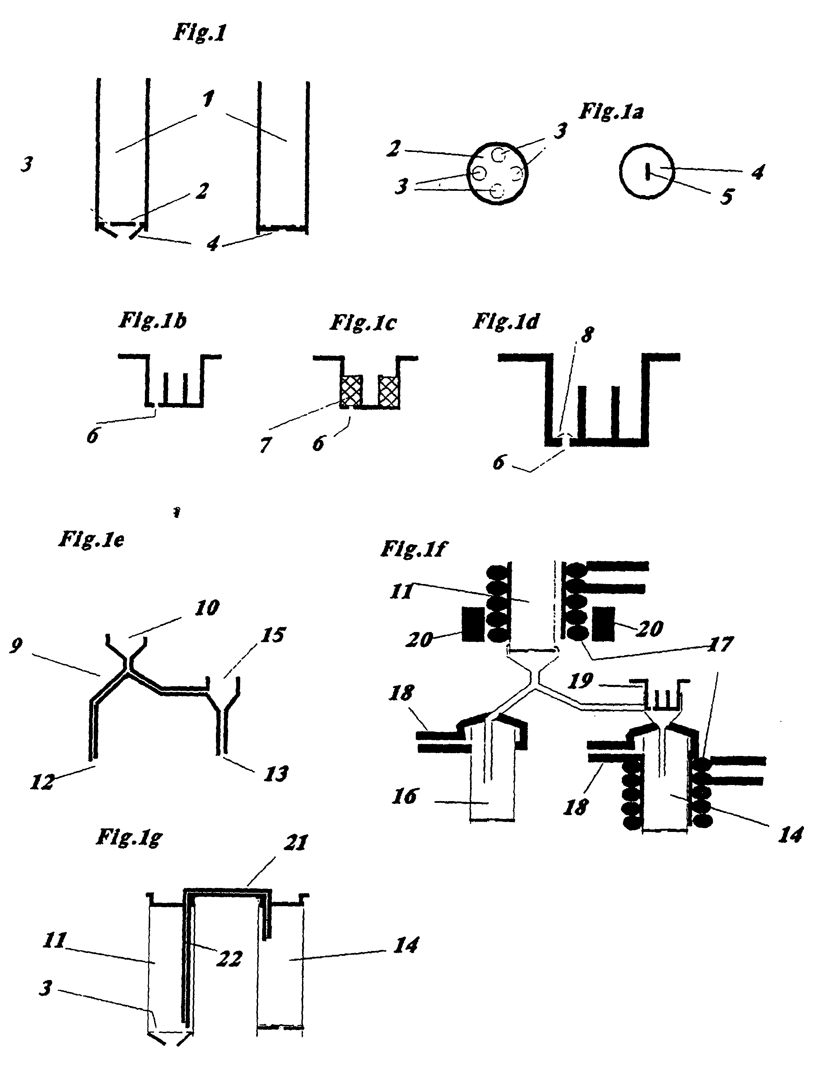

In Figur 1 sind Module gezeigt, die für die Herstellung einer erfindungsgemäßen Vorrichtung verwendet werden können und zeigt ein Universalmodul (1) fiir Reaktionsräume mit einer verschließbaren Bodenöffnung im geöffneten und geschlossenen Zustand. In einer bevorzugten Ausführungsform besteht das Universalmodul aus einem Boden (2) mit mehreren Auslaßöffnungen (3) und einer geschlitzten Gummidichtlippe (4).

Fig. 1a zeigt die Aufsicht auf Boden (2) mit Auslaßöffnungen (3) sowie Gummidichtlippe (4) mit Schlitz (5). Durch Anlegen von Unterdruck wölbt sich die Gummidichtlippe (4), öffnet den Schlitz (5) und gibt die Bodenöffnungen (3) frei.

Fig. 1b zeigt den Schnitt durch den Deckel für die obere Öffnung mit Belüftungsloch (6). Die Ausführungen Fig. 1c und 1d berücksichtigen den Kontaminationsschutz, wobei in Fig. 1c eine Papiervliesscheibe (7) und in Fig. 1d eine geschlitzte Gummidichtlippe (8) verwendet wird.

Figur 1e zeigt ein Verteilerelement (9) mit einem Ansatz (10) für einen ersten Reaktionsraum (11), einem Ansatz (12) für einen Abfallbehälter und einen Ansatz (13) für ein zweites Modul mit einem zweiten Reaktionsraum (14) in einer Kaskadenanordnung. Die Öffnung (15) dient zur Zugabe von Reagenzien und Aufnahme eines Deckels (19).

In Figur 1f ist eine erfindungsgemäße Vorrichtung mit einem ersten Reaktionsraum (11), einem zweiten Reaktionsraum (14), einem Abfallbehälter (16), Heizelementen (17) für den ersten und zweiten Reaktionsraum sowie Vakuumansatzstutzen (18) für den zweiten Reaktionsraum und den Abfallbehälter gezeigt. Im gezeigten Zustand ist auf dem Verteilerelement in der Nähe des zweiten Reaktionsraums ein Deckel (19) vorgesehen, durch welchen die Reagenzien für die Vermehrung der Nukleinsäuren in den zweiten Reaktionsraum gegeben werden können. Ferner ist ein Magnet (20) angedeutet, der zur Separierung magnetischer Phasen (z. B. Partikel von nicht magnetischen Phasen) während oder nach der Vermehrungsreaktion benutzt werden kann z. B. für eine vorgeschaltete Zellanreicherung mittels Immunadsorbtion.

Eine nicht unter die unabhängigen Ansprüche fallende planare Anordnung zeigt Fig. 1g. Das Verteilerelement (21) ist hier in die Deckellösung integriert. Über eine Kapillare (22) kann die Reaktionslösung vom ersten Reaktionsraum (11) in den zweiten (14) durch Unterdruck im zweiten Reaktionsraum (14) bei geschlossenem Ventil im ersten Reaktionsraum (11) überführt werden. Bei den diversen Reaktionen anfallender Abfall kann dabei direkt über die Bodenöffnungen (3) entfernt werden. Analog Fig. 1f können Heizelemente und Magnetseparator eingesetzt werden.

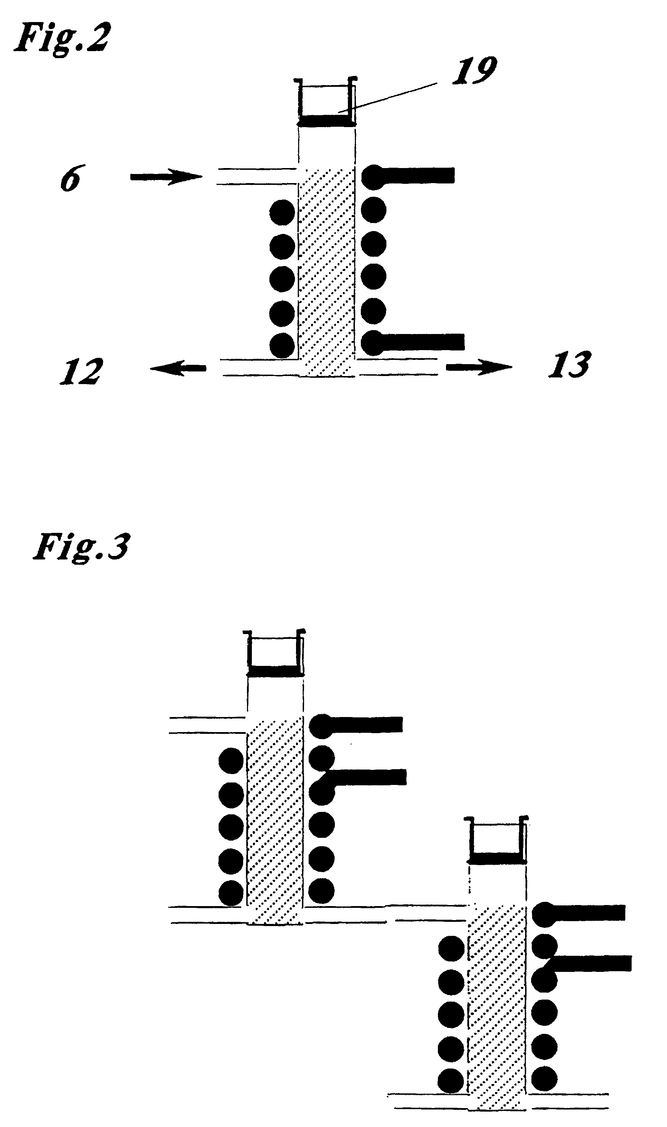

In Figur 2 ist ein anderes Beispiel für den variablen Aufbau eines kontaminationsfreien Reaktionsraumes aus Standardmodulen gezeigt, der zur immunologischen Adsorption von Zellen genutzt werden kann. Im gezeigten Zustand ist das Modul durch einen Deckel (19) verschlossen. Bei Anlegen eines Vakuums an den Anschlüssen (12,13) würde ein Transport der Flüssigkeit aus dem Modul schnell zum Erliegen kommen, wenn nicht eine weitere Öffnung (6) zum Druckausgleich vorgesehen wäre.

In Figur 3 ist die Kombination eines Moduls mit Reagenzien zur Immunadsorption mit einem Modul für die Durchführung einer Vermehrungsreaktion von Nukleinsäuren schematisch gezeigt. Es wird klar, daß, sofern die Übergänge aufeinander abgestimmt sind, die Module für jeden Behandlungsschritt adaptiert und mit entsprechenden Heizelementen oder Magnetseparatoren versehen werden können und so analog Fig. 1a-g ein universelles System aus einfachen Grundmodulen ergeben.

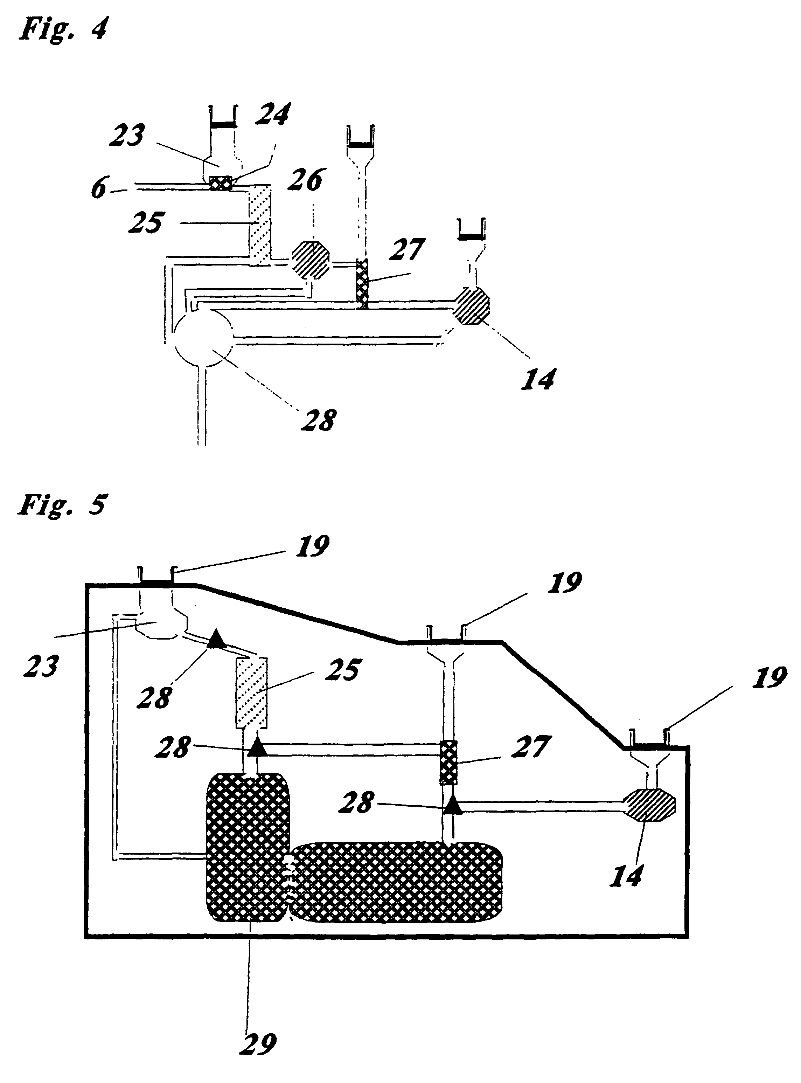

In Figur 4 ist eine erfindungsgemäße Vorrichtung zur Probenvorbereitung und Amplifikation von Nukleinsäuren aus Vollblut dargestellt. In einem Reaktionsmodul (23) findet die Hämolyse statt. Die gewünschten Zellen werden über ein Grobfilter (24) von Geweberesten befreit. Durch Druckausgleich über den Ausgang (6) werden die Zellen auf einen Immunadsorber (25) aufgegeben. Durch Elution mit Puffern im pH-Bereich 2-3 werden die Zellen eluiert und die Suspension in einen Reaktionsraum (26) überführt, in dem die Lyse der Zellen stattfindet. Das gesamte Lysat wird in ein Modul (27) überführt, welches ein Glasvlies enthält. Daran werden die Nukleinsäuren adsobiert, während die anderen Probenbestandteile im Filtrat verbleiben und dem Abfall zugeführt werden. Nach Desorption mittels Puffer mit niedrigen Ionenstärken wird die nukleinsäurehaltige Flüssigkeit in eine Amplifikationskammer (14) überführt.

Der Transport von Flüssigkeiten wird in dem hier beschriebenen Device mittels eines einzigen Mehrwegeventils (28) und Anlegen von Unterdruck sowie der Belüftung (6) bewerkstelligt

In Figur 5 ist ein einteiliges Device für die Bearbeitung von EDTA-Blut gezeigt. Der Flüssigkeitstransport findet über ein Abfallvlies (29) (Kapillarität zusammen mit Erdanziehungskraft) statt. In einem ersten Gefäß (23) findet eine Preinkubation, wie z. B. enzymatische Verflüssigung oder die oben erwähnte Hämolyse statt. Es schließt sich ein Immunadsorber (25) an. Die Nukleinsäuren werden über ein anschließendes Glasvlies (27) immobilisiert und von anderen Probenbestandteilen abgetrennt. Anschließend wird die nukleinsäurehaltige Lösung in die Amplifikationskammer (14) überführt. Im vorliegenden Fall sind die Verteilerelemente (28) 3-Wege-Hähne.

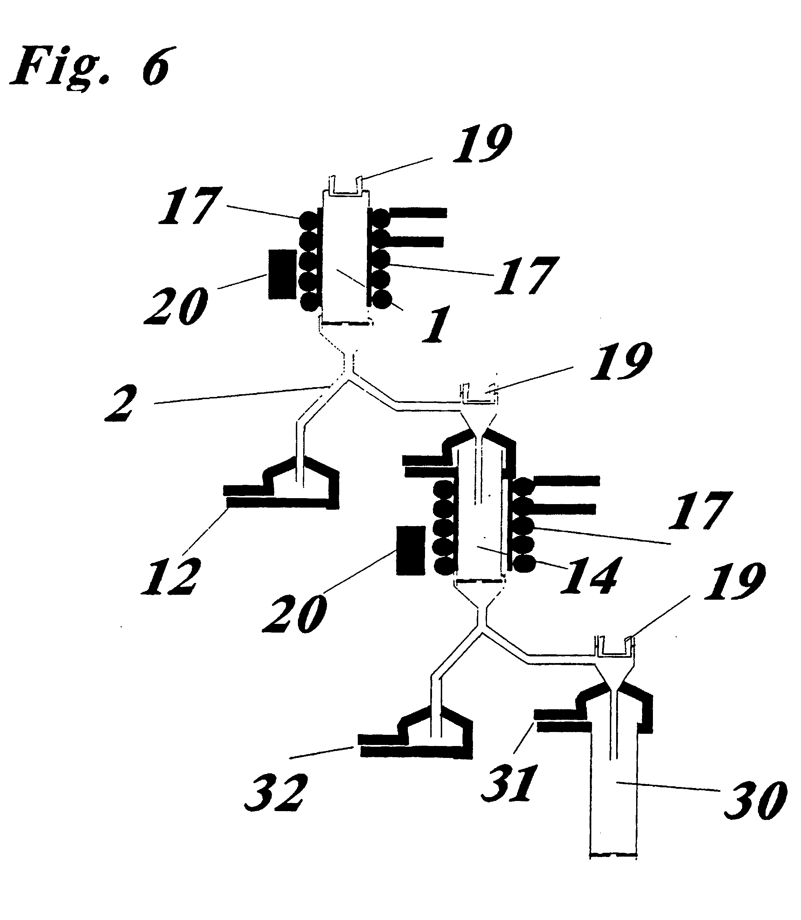

In Figur 6 ist ein Schema für eine Aufbereitung von HIV-haltigem Vollblut für einen Nachweis der DNA oder RNA gezeigt. Es wird hierbei auf die Beschreibung von Figur 1c verwiesen. Zusätzlich ist ein Magnet (20) auch im ersten Modul vorgesehen. Darüber hinaus ist angedeutet, daß die amplifizierten Nukleinsäuren in ein konventionelles Tube (30) überführt und nachgewiesen werden können. Diese Figur ist in Beispiel 1 näher beschrieben.

Ebenfalls Gegenstand der Erfindung ist ein System zur Behandlung von Nukleinsäuren enthaltend eine Vorrichtung zum Pipettieren von Flüssigkeiten, eine Vorrichtung zur Aufnahme einer Vorrichtung gemäß Anspruch 12, eine Vorrichtung gemäß Anspruch 12 und eine Vorrichtung zum geregelten Transport von Gasen oder Flüssigkeiten in der Vorrichtung gemäß Anspruch 12. Als Vorrichtung zum Pipettieren von Flüssigkeiten können übliche Pipettierautomaten z. B. der Firma Tecan verwendet werden. Mit diesen Automaten können sowohl die nukleinsäurehaltige Probeflüssigkeit als auch eventuell erforderliche Reagenzien aus Proben- bzw. Reagenzvorratsgefäßen in die erfindungsgemäße Vorrichtung überführt werden. Der Transport in der erfindungsgemäßen Vorrichtung wird bevorzugt über das Anlegen von Unterdruck gesteuert. Hierzu wird eine Anschlußöffnung der Vorrichtung mit einem Element verbunden, mit dem ein Unterdruck erzeugt werden kann. Daß die Flüssigkeiten aus den einzelnen Reaktionsräumen wie gewünscht weiter transportiert werden, kann über eine Steuerung der Verteilerelemente oder/und der Unterdruckanlage gewährleistet werden.

Ebenfalls Gegenstand der Erfindung ist ein Verfahren zur Abtrennung und Vermehrung von Nukleinsäuren aus einer Probe durch
- Immobilisierung der Nukleinsäuren in einem ersten Reaktionsraum,
- Entfernung anderer Probenteile von den immobilisierten Nukleinsäuren über einen regelbaren Transportweg,
- die Immobilisierung der Nukleinsäuren und Überführung der nukleinsäurehaltigen Flüssigkeit über einen regelbaren Transportweg in einen zweiten Reaktionsraum,
- Amplifikation der Nukleinsäuren im zweiten Reaktionsraum, nach Anspruch 1.

Vorteil der Erfindung ist die hohe Flexibilität des Einsatzes der Module, die Möglichkeit, die Kontaminationen gering zu halten und die Automatisierbarkeit von Probenvorbereitung und Amplifikation bei nukleinsäurediagnostischen Tests. Die Module sind bevorzugt so aufgebaut, daß sie nach einmaliger Benutzung verworfen werden können. Der jedoch wohl größte Vorteil der Erfindung ist die Möglichkeit, in den Schritten Probenvorbereitung und Amplifikation jegliche Zentrifugation vermeiden zu können.

Die folgenden Beispiele sollen die Erfindung näher erläutern:

### Beispiel 1a:

### Bestimmung von HIV-DNA aus Vollblut (FIG 6)

Im Reaktionsmodul (1) werden mittels biotinyliertem Anti-T_{Helfer}-Zellen/CD4, human-Antikörper (Hersteller: Boehringer Mannheim Biochemica Kat.Nr.: 881 171) und Streptavidin-Magnet-Partikeln (Hersteller: Dynal, Oslo, Norwegen) die HIV-DNA-haltigen Zellen aus Vollblut suspendiert, inkubiert und über einen ersten Magneten (20) zurückgehalten. Durch Anlegen von Vakuum an den Abfallbehälter (16) über den Stutzen (18) kann das nicht benötigte Vollblut ausgewaschen und entfernt werden.

Durch Zugabe von Proteinase K in einem geeigneten Puffersystem z.B. mit 1% Laureth 12 oder 0,5 % Tween 20 nach Rolfs et al. (in PCR: Clinical Diagnostics and Research, Springer Verlag, 1992) S. 84 ff oder mit Natrium-Dodecylsulfatzusatz nach Anderson in Diagnostic Molecular Microbiology - Principals and Applications Edtrs.: D.H. Persing, T.F. Smith, F.C. Tenover und T.J. White; American Society of Microbiology; ISBN 1-55581-056-X (1993) S. 197-202 können die Zellen nach Resuspension durch Erhitzen über die erste Heizung (17) lysiert werden. Nach Zurückhalten der magnetischen Partikel durch Magnet (20) und durch Anlagen von Vakuum an (18) kann die Reaktionsmischung in Reaktionsmodul (14) überführt werden.

Durch den automatisch zu öffnenden Deckel (19) können anschließend PCR-Reagenzien zugegeben und durch Thermocycling über die Heizung (17) des Moduls (14) kann die DNA amplifiziert werden analog zu EP-A-324474 . Danach wird durch Zugabe von biotinylierter DNA-capture-Probe, die zu dem PCR-Amplifikat zwischen den Primerhybridisierungspositionen komplementär ist, und Erhitzen über die Heizung (17) des Moduls (14) eine Hybridisierung durchgeführt werden. Anschließend kann durch Vakuumanlegen an (31) die gesamte Reaktionsmischung in ein Streptavidin(SA)-beschichtetes (ES) Tube (30, Boehringer Mannheim GmbH, Best.-Nr. 1602845) gefüllt in einem ES-Gerät (Boehringer Mannheim GmbH, Best.-Nr. 1602845) detektiert und das Ergebnis ausgewertet werden.

### Beispiel 1b:

In einer Variante kann stattdessen in einem 2-Stufen-Prozeß statt PCR-Reagenz nach EP-A-0 389 063 Silicagel mit GuSCN-Puffer zugegeben werden, der die DNA unspezifisch adsorbiert. Überschüssiges Material kann durch Anlegen von Vakuum an (32) abgetrennt werden, falls zuvor eine entsprechende Filterschicht in (14) eingelegt wurde. Elution der Nukleinsäuren vom Silicagel erfolgt durch niedrig Salzpuffer (10 mM Tris-Hydrochlorid-1mM EDTA (pH8.0) nach Boom et al. J. of. Clinical Microbiology, Vol. 28, Nr. 3; 1990; S. 496) mit anschließender PCR in einem weiteren, zwischengeschalteten Modul, Hybridisierung und Detektion (wie in 1a).

### Beispiel 2:

### Bestimmung von HIV-RNA aus Vollblut (FIG 6)

In (1) wird Vollblut einer Hämolyse nach Rolfs et al. (in PCR: Clinical Diagnostics and Research, Springer Verlag, 1992) S. 69 - 70 unterworfen. Anschließend werden durch Zugabe von biotinyliertem Anti-gp 120-Antikörper vom Schaf (Hersteller: Aalto Bio Reagents Ltd. Dublin, Irland) Viruspartikel über SA-beschichtete magnetische Beads (Hersteller: siehe Beispiel 1) abgetrennt und gemäß Beispiel 1a oder 1b aufgearbeitet, wobei statt PCR zur Amplifikation von RNA eine RT-PCR analog zu Wilber et al. in Diagnostic Molecular Microbiology - Principals and Applications Edtrs.: D.H. Persing, T.F. Smith, F.C. Tenover und T.J. White; American Society of Microbiology; ISBN 1-55581-056-X (1993) S. 327-331 angewendet werden muß.

### Beispiel 3:

### Verarbeitung von Gewebe

Gewebe wird analog der von Lewin et al. in "Methods in Enzymology" Vol. 171 "Biomembranes"; Edtrs: S. Fleischer und B. Fleischer; Academic Press; S. 444 ff nach der Pronase EDTA-Methode oder der Collagenase-Pronase-EDTA Methode durch Erhitzen mittels Heizung (17) auf 37 Grad C in Reaktionsmodul (1) in Einzelzellen übergeführt. Mittels spezifischer biotinylierter Zelloberflächenmarker analog zu Garcia-Perez et al. in "Methods in Enzymology" Vol. 171 "Biomembranes"; Edtrs: S. Fleischer und B. Fleischer; Academic Press; S. 581 und Magnetpartikel (Hersteller sh. Besipiel 1) werden spezifische Zellen isoliert und gereinigt, die gemäß Beispiel 1a oder 1b der PCR zugeführt werden.

### Beispiel 4:

### Aufschluß von Sputum zur Diagnose von Mycobacterien

Sputum wird zur Verflüssigung nach der Methode, beschrieben bei Rolfs et al. (in PCR: Clinical Diagnostics and Research, Springer Verlag, 1992) S. 74 ff, in das Reaktionsmodul (1) und mit N-Acetyl-L-Cystein/NaOH-Lösung gegeben, wobei durch leichtes Erhitzen durch das Heizelement (17) die Reaktion beschleunigt werden kann. Die in der Originalliteratur angegebenen Zentrifugationsschritte werden ersetzt durch Einsatz spezifischer biotinylierter Antikörper gegen Oberflächenmarker von Mycobacterium hergestellt analog zu Garcia-Perez et al. in "Methods in Enzymology" Vol. 171 "Biomembranes"; Edtrs: S. Fleischer und B. Fleischer; Academic Press; S. 581 und Streptavidin beschichteten Magnetpartikel (Hersteller sh. Beispiel 1). Spezifische Zellen werden isoliert und wie in Beispiel 3 weiterbehandelt.

### Beispiel 5:

### Einsatz von Säulenchromatographie für die Zellisolierung bei HTV-DNA-Nachweis

Reaktionsmodul (1) wird vor Zugabe der HIV-haltigen Probe befüllt mit Streptavidin-gekoppelter Bromcyan-aktivierter Sepharose (Hersteller Streptavidin: Boehringer Mannheim 1097679; Verfahren beschrieben in "Immunchemische Charakterisierung der ATP-Synthetase von E. coli K12", G. Bienhaus, Dissertation Universität Tübingen, S. 66, 1980). Dazu werden Reagenzien analog Beispiel 1a gegeben. Nach Reaktion wird überschüssiges Material durch Anlegen von Vakuum an (4) abgetrennt, falls eine entsprechende Filterschicht in (1) zuvor eingelegt wurde.

### Beispiel 6:

### Einsatz von NASBA statt PCR

Statt der PCR kann auch das NASBA-Verfahren (Cangene USP 5 130 239) angewendet werden, wobei über die Heizung (17) des Moduls (14) eine konstante Temperatur erzeugt werden kann.

### Beispiel 7:

### Einsatz von Ionenaustauschchromatographie anstatt Glasvlies

Statt der Variante 1b kann auch gemäß DE 41 39 664 eine Ionenaustauschchromatographie zur Isolierung von Nukleinsäuren benutzt werden.

### Bezugszeichenliste

- 1: Reaktionsmodul
- 2: Boden von 1
- 3: Auslaßöffnungen in 2
- 4: Gummidichtlippe
- 5: Schlitz in 4
- 6: Belüftungsloch in 1
- 7: Papiervliesscheibe
- 8: geschlitzte Gummidichtlippe im Deckel
- 9: Verteilerelement
- 10: Ansatz
- 11: erster Reaktionsraum
- 12: Ansatz für Abfallbehälter
- 13: Ansatz für zweites Modul
- 14: Reaktionsraum des Amplifikationsmoduls
- 15: Öffnung des zweiten Moduls
- 16: Abfallbehälter
- 17: Heizelement der Module
- 18: Vakuumansatzstutzen
- 19: Deckel
- 20: Magnet
- 21: Verteilerlement einer planaren Anordnung
- 22: Kapillare
- 23: Reaktionsmodul für Hämolyse
- 24: Grobfilter
- 25: Immunadsorber
- 26: Reaktionsraum für Lyse
- 27: Modul für Adsorption von Nukleinsäuren
- 28: Mehrwegeventil
- 29: Abfallvlies
- 30: Tube
- 31: Vakuumansatzstutzen an Tubehalter
- 32: Vakuumansatzstutzen an Abfallbehälter

## Patentansprüche

1. Verfahren zur Behandlung von Nukleinsäuren aus einer Probe in einer einen ersten und einen zweiten Reaktionsraum enthaltenden Vorrichtung, wobei
- die Nukleinsäuren aus der Probe an Magnetpartikel gebunden werden,
- Probenbestandteile, die in Vermehrungsprozessen inhibitorisch wirken, von den gebundenen Nukleinsäuren abgetrennt werden,
- die Nukleinsäuren wieder in Lösung gebracht werden,
- die die Nukleinsäuren enthaltende Lösung in den zweiten Reaktionsraum überführt wird, wobei die Überführung der Lösung über einen Transportweg zwischen dem ersten und zweiten Reaktionsraum geschieht, wobei der Transportweg so regelbar ist, daß nur die die Nukleinsäuren enthaltende Lösung, nicht jedoch eine mit den anderen Probenbestandteilen beladene Flüssigkeit in den zweiten Reaktionsraum gelangt und dieser Transportweg ein 3-Wege-Verteilerelement enthält, von dem jeweils ein Weg zu dem ersten und zweiten Reaktionsraum führt und ein dritter Weg ein Abfalltransportweg ist, und
- Vermehrung der Nukleinsäuren in dem zweiten Reaktionsraum.

2. Verfahren gemäß Anspruch 1, **dadurch gekennzeichnet, daß** die Vorrichtung ein geschlossenes System darstellt, bei dem jeweils im ersten und zweiten Reaktionsraum verschließbare Öffnungen vorgesehen sind.

3. Verfahren gemäß Anspruch 1, **dadurch gekennzeichnet, daß** im ersten Reaktionsraum auch eine Verflüssigung der Probe oder eine Freisetzung von Nukleinsäuren oder einer Kombination davon stattfindet.

4. Verfahren gemäß Anspruch 1, **dadurch gekennzeichnet, daß** der zweite Reaktionsraum eine Öffnung zur Entfernung der Reaktionsmischung aus dem Reaktionsraum aufweist.

5. Verfahren gemäß Anspruch 1, **dadurch gekennzeichnet, daß** mindestens ein Reaktionsraum beheizbar ist.

6. Verfahren gemäß Anspruch 1, **dadurch gekennzeichnet, daß** der zweite Reaktionsraum eine verschließbare Öffnung zur Zugabe von Reagenzien für eine Vermehrungsreaktion aufweist.

7. Verfahren gemäß Anspruch 2, **dadurch gekennzeichnet, daß** das geschlossene System aus Einwegmodulen besteht.

8. Verfahren gemäß Anspruch 2, **dadurch gekennzeichnet, daß** das geschlossene System aus mehreren im wesentlichen baugleichen, zusammensteckbaren Modulen besteht, die mehrstufige Reaktionsabfolgen ermöglichen.

9. Verfahren gemäß Anspruch 1, **dadurch gekennzeichnet, daß** mindestens ein Reaktionsraum mittels Unterdruck entleert werden kann.

10. Verfahren gemäß Anspruch 1, **dadurch gekennzeichnet, daß** mindestens ein Reaktionsraum mit einem Magnetseparator zur Trennung magnetischer und nicht-magnetischer Phasen versehen ist.

11. Verfahren gemäß Anspruch 1, **dadurch gekennzeichnet, daß** die Vorrichtung als anwendungsbereites Mehrfachreaktionsgefäß verwendet wird.

12. Vorrichtung zur Behandlung von Nukleinsäuren aus einer Probe, **dadurch gekennzeichnet, daß** sie einen ersten Reaktionsraum mit einem Magnetseparator zur Abtrennung von Nukleinsäuren von Probenbestandteilen, die in Vermehrungsprozessen inhibitorisch wirken, sowie einen damit über einen Transportweg verbundenen zweiten Reaktionsraum zur Vermehrung der Nukleinsäuren enthält, wobei der Transportweg so regelbar ist, daß nur eine die Nukleinsäuren enthaltende Lösung, nicht jedoch eine mit den anderen Probenbestandteilen beladene Flüssigkeit in den zweiten Reaktionsraum gelangt und dieser Transportweg ein 3-Wege-Verteilerelement enthält, von dem jeweils ein Weg zu dem ersten und zweiten Reaktionsraum führt und ein dritter Weg ein Abfalltransportweg ist.

13. System zur Behandlung von Nukleinsäuren enthaltend
- eine Vorrichtung zum Pipettieren von Flüssigkeiten
- eine Vorrichtung zur Aufnahme einer Vorrichtung gemäß Anspruch 12
- eine Vorrichtung gemäß Anspruch 12 und
- eine Vorrichtung zum geregelten Transport von Gasen oder Flüssigkeiten in der Vorrichtung gemäß Anspruch 12.

14. System gemäß Anspruch 13, **dadurch gekennzeichnet, daß** es eine Vorrichtung zum Erhitzen eines oder mehrerer der Reaktionsräume der Vorrichtung des Anspruchs 12 enthält.

15. System gemäß Anspruch 14, **dadurch gekennzeichnet, daß** es einen oder mehrere Magneten enthält, die in der Nähe des ersten und/oder zweiten Reaktionsraumes positioniert werden können.

## Claims

1. Method for treating nucleic acids from a sample in an apparatus comprising a first and a second reaction chamber, wherein
- the nucleic acids from the sample are bound to magnetic particles,
- sample components which inhibit amplification processes are separated from the bound nucleic acids,
- the nucleic acids are redissolved,
- the solution containing the nucleic acids is transferred to the second reaction chamber, the transfer of the solution occurring by means of a transport path between the first and second reaction chamber, wherein the transport path can be controlled such that only the solution containing the nucleic acids but not a liquid loaded with other sample components reaches the second reaction chamber and this transport path contains a three-way distributing element from which one path leads to the third reaction chamber and one path leads to the second reaction chamber and a third path is for transporting waste and
- amplifying the nucleic acids in the second reaction chamber.

2. Method as claimed in claim 1, wherein the apparatus is a closed system in which closable openings are provided in the first and second reaction chamber.

3. Method as claimed in claim 1, wherein the sample is liquefied or nucleic acids are released or a combination of both takes place in the first reaction chamber.

4. Method as claimed in claim 1, wherein the second reaction chamber has an opening to remove the reaction mixture from the reaction chamber.

5. Method as claimed in claim 1, wherein at least one reaction chamber can be heated.

6. method as claimed in claim 1, wherein the second reaction chamber has a closable opening for adding reagents for an amplification reaction.

7. Method as claimed in claim 2, wherein the closed system is composed of disposable modules.

8. Method as claimed in claim 2, wherein the closed system is composed of several modules that can be plugged together and have essentially the same construction which enable multistep reaction sequences.

9. Method as claimed in claim 1, wherein at least one reaction chamber can be emptied by means of negative pressure.

10. Method as claimed in claim 1, wherein at least one reaction chamber is provided with a magnetic separator in order to separate magnetic and nonmagnetic phases.

11. Method as claimed in claim 1, wherein the apparatus is used as a ready-to-use multireaction vessel.

12. Apparatus for treating nucleic acids from a sample, wherein it comprises a first reaction chamber having a magnetic separator to separate nucleic acids from sample components which inhibit amplification processes and a second reaction chamber for amplifying nucleic acids which is connected thereto by means of a transport path, the transport path being controllable in such a manner that only a solution containing the nucleic acids but not a liquid loaded with other sample components can reach the second reaction chamber and this transport path contains a three-way distributing element one path of which leads to the first reaction chamber and one path of which leads to the second reaction chamber and a third path is a waste disposable path.

13. System for treating nucleic acids comprising
- a device for pipetting liquids
- a device for holding an apparatus as claimed in claim 12
- an apparatus as claimed in claim 12
and
- a device for the controlled transport of gases or liquids in the apparatus as claimed in claim 12.

14. System as claimed in claim 13, wherein it contains a device for heating one or more of the reaction chambers of the apparatus of claim 12.

15. System as claimed in claim 14, wherein it contains one or more magnets which can be positioned near to the first and/or second reaction chamber.

## Revendications

1. Procédé pour le traitement d'acides nucléiques dans un échantillon dans un dispositif contenant une première et une deuxième chambre de réaction,
- les acides nucléiques de l'échantillon étant fixés à des particules magnétiques,
- les constituants de l'échantillon qui ont un effet d'inhibition dans les processus de multiplication étant séparés des acides nucléiques fixés,
- les acides nucléiques étant à nouveau amenés en solution,
- la solution contenant les acides nucléiques étant transvasée dans la deuxième chambre de réaction, le transvasement de la solution étant réalisé via une voie de transport entre la première et la deuxième chambre de réaction, la voie de transport étant réglable de telle manière que seule la solution contenant les acides nucléiques, et non pas un liquide chargé avec les autres constituants de l'échantillon, arrive dans la deuxième chambre de réaction et cette voie de transport contenant un élément de répartition à trois voies, dont à chaque fois une voie conduit à la première chambre de réaction et à la deuxième chambre de réaction et la troisième voie est une voie de transport des déchets et
- multiplication des acides nucléiques dans la deuxième chambre de réaction.

2. Procédé selon la revendication 1, **caractérisé en ce que** le dispositif représente un système fermé, dans lequel des ouvertures pouvant être fermées sont à chaque fois prévues dans la première et la deuxième chambre de réaction.

3. Procédé selon la revendication 1, **caractérisé en ce qu'**une liquéfaction de l'échantillon ou une libération d'acides nucléiques ou une combinaison de celle-ci se produit également dans la première chambre de réaction.

4. Procédé selon la revendication 1, **caractérisé en ce que** la deuxième chambre de réaction présente une ouverture pour l'élimination du mélange réactionnel hors de la chambre de réaction.

5. Procédé selon la revendication 1, **caractérisé en ce qu'**au moins une chambre de réaction peut être chauffée.

6. Procédé selon la revendication 1, **caractérisé en ce que** la deuxième chambre de réaction présente une ouverture pouvant être fermée pour l'addition de réactifs pour une réaction de multiplication.

7. Procédé selon la revendication 2, **caractérisé en ce que** le système fermé est constitué de modules à voie unique.

8. Procédé selon la revendication 2, **caractérisé en ce que** le système fermé est constitué de plusieurs modules, de construction essentiellement identique, pouvant être assemblés, qui permettent des déroulements de réaction en plusieurs étapes.

9. Procédé selon la revendication 1, **caractérisé en ce qu'**au moins une chambre de réaction peut être vidée au moyen d'une dépression.

10. Procédé selon la revendication 1, **caractérisé en ce qu'**au moins une chambre de réaction est pourvue d'un séparateur magnétique pour la séparation de phases magnétiques ou non magnétiques.

11. Procédé selon la revendication 1, **caractérisé en ce que** le dispositif est utilisé comme récipient à réactions multiples prêt à l'emploi.

12. Dispositif pour le traitement d'acides nucléiques d'un échantillon, **caractérisé en ce qu'**il contient une première chambre de réaction avec un séparateur magnétique pour la séparation des acides nucléiques des constituants de l'échantillon, qui ont un effet inhibiteur dans des processus de multiplication, ainsi qu'une deuxième chambre de réaction, reliée à ladite première chambre via une voie de transport, pour la multiplication des acides nucléiques, la voie de transport étant réglable de telle manière que seule la solution contenant les acides nucléiques, et non pas un liquide chargé des autres constituants de l'échantillon, arrive dans la deuxième chambre de réaction et cette voie de transport contenant un élément de répartition à trois voies, dont à chaque fois une voie conduit à la première et à la deuxième chambre de transport et la troisième chambre de transport est une voie de transport des déchets.

13. Système pour le traitement d'acides nucléiques contenant :
- un dispositif pour pipeter des liquides,
- un dispositif destiné à recevoir un dispositif selon la revendication 12,
- un dispositif selon la revendication 12 et
- un dispositif pour le transport réglé de gaz ou de liquides dans le dispositif selon la revendication 12.

14. Système selon la revendication 13, **caractérisé en ce qu'**il contient un dispositif pour le chauffage d'une ou de plusieurs chambres de réaction du dispositif de la revendication 12.

15. Système selon la revendication 14, **caractérisé en ce qu'**il contient un ou plusieurs aimants qui peuvent être positionnés à proximité de la première et/ou de la deuxième chambre de réaction.
